Europäisches Patentamt

European Patent Office

Office européen des brevets

(11) Publication number: **0 405 598 A2**

# EUROPEAN PATENT APPLICATION

(21) Application number: 90112493.3

(22) Date of filing: 29.06.90

(51) Int. Cl.5: **A61K 7/34, A61K 7/38,**
C01F 7/48, C01G 25/04

(30) Priority: 30.06.89 US 374383

(43) Date of publication of application:
02.01.91 Bulletin 91/01

(84) Designated Contracting States:
DE FR GB

(71) Applicant: DOW CORNING CORPORATION
P.O. Box 1767
Midland Michigan 48686-0994(US)

(72) Inventor: Gatti, Christopher Darren
P.O. Box 1767
Montague, New Jersey 07827(US)
Inventor: Buzzelli, Anthony John
10 Colvert Street, Apt. 2
Port Jervis, New York(US)
Inventor: Liscomb, Cristina Birgitta
137 Lakeview Drive
Highland Lake, New York(US)
Inventor: Abrutyn, Eric Steven
301 Chapel Lane
Midland, Michigan(US)
Inventor: Rentsch, Stefan Felix
5800 Siebert Street
Midland, Michigan(US)
Inventor: Sloan, Robert James
57 K Village Green
Budd Lake, New Jersey(US)

(74) Representative: Spott, Gottfried, Dr. et al
Patentanwälte Spott und Puschmann
Sendlinger-Tor-Platz 11
D-8000 München 2(DE)

(54) Process to control polymer distribution in antiperspirant salts.

(57) This invention relates to a method of forming activated antiperspirant salts, wherein the activated salts are formed by heating a solution of $Z_2(OH)_{6-a}X_a$, wherein Z is a member selected from the group consisting of aluminum, zirconium and mixtures thereof, a ranges from 0.3 to 4 and X is a member selected from the group consisting of Cl, Br, I and mixtures thereof, to a temperature from about 140° to about 200°C. for a period of time ranging from one to five minutes or one to thirty minutes if a recycling loop heat exchanger is used and then drying said salt solution in its activated state. This process provides a much more rapid means of making activated antiperspirant salts, as it is taught in the art to heat the starting materials for periods up to thirty days.

EP 0 405 598 A2

# PROCESS TO CONTROL POLYMER DISTRIBUTION IN ANTIPERSPIRANT SALTS

This invention relates to a process for forming activated antiperspirant salts.

It has long been known that certain astringent salts, for example, aluminum and zirconium, and in particular, aluminum and zirconium halohydrate salts, such as aluminum chlorhydrate, zirconium chlorhydrate and aluminum/zirconium chlorhydrate complexes, act as antiperspirants when applied to the human body. Although these materials provide a measure of protection against perspiration, research in the personal care industry has been directed at improving their efficacy as antiperspirants.

Antiperspirant salts are polymeric in nature due to the aluminum and zirconium base metals of the salts being polyvalent. As a general proposition, polyvalent elements, and in particular, aluminum and zirconium, are capable to forming compounds having a polymeric structure. Aluminum and zirconium compounds normally exist in a polymeric state, except when exposed to highly acidic environments.

Antiperspirant salts normally comprise a mixture of salt species based on the various polymeric states in which the salts exist. Differences in the size, molecular weight and molecular structure of the polymers readily enable one to distinguish the various antiperspirant polymer species. The molecular size, weight and structure of the various species and the relative amounts of the various species of aluminum polymers and zirconium polymers presnet in a typical antiperspirant salt sample are dependent on the methods and conditions used in processing the antiperspirant salt. The inventors have found that in making antiperspirant salts, the concentration of the materials, pH and temperature impact the types and relative proportions of the various species.

Certain antiperspirant salt species exhibit high antiperspirant efficacy. It is recognized by the industry that when a high efficacy species is present in a salt in sufficient quantities, the salt is referred to as an "activated antiperspirant salt", "enhanced antiperspirant salt", "super antiperspirant salt" or as preferred by the inventors, an "activated salt".

Experimentation has lead to the discovery that antiperspirant salt processing techniques can be practiced which favor the formation of the higher efficacy species. The resulting activated salts in which these species are contained are generally recognized as having high commercial value.

United States Patent No. 4,359,456 describes a process for making an aluminum chlorhydrate salt having a high concentration of the higher efficacy species. The process described in the '456 patent comprises heating an aqueous solution of $Al_2(OH)_{6-a}X_a$, wherein a ranges from 0.3 to 4 and X represents Cl, Br or I at temperature of from 50° to 140°C. for a period of time ranging from one half hour to thirty days. The resulting salt solution is allowed to cool to room temperature and is then spray dried to obtain an activated antiperspirant salt powder.

Research has lead to other processes for obtaining antiperspirant salt materials with high levels of activated components. For example, European Patent Application 0 256 831 relates to a batch process for the preparation of activated aluminum and zirconium halohydrates. The process of the '831 application requires that the mixture of antiperspirant salt forming materials be held at a temperature of

"... about 50° C. - 105° C. for a time just long enough to dissolve sufficient aluminum to produce an aqueous solution of a final aluminum zirconium halohydrate having an aluminum:zirconium molar ratio in the range 2:1 to 7:1 and a metal:halogen molar ratio of from 0.9:1 to 2.1:1 ... "

According to the examples in the '831 application, the time required to "dissolve sufficient aluminum" is rather lengthy, ranging from three hours to five days.

European Patent Application 0 256 832 regards the same general method for the preparation of antiperspirant salts with a high concentration of activated components as disclosed in the '831 application. The '832 application is important for teaching that the production of the activated species is strongly influenced by the reaction temperature and that lower reaction temperatures favor the formation of activated salts.

Additionally, European Patent Application 0 191 628 relates to the preparation of basic aluminum halides. The '682 application is generally related to the '831 and '832 patent applications.

The examples presented in the patents and published applications referred to above teach that the heating of the raw antiperspirant material be carried out at temperatures up to 140°C. with lower temperatures preferred and that the heating step be an extended period of time lasting from one half hour to thirty days. Furthermore, the examples of the patents and applications named above indicate that the preparation of the activated salts can only be carried out as a batch process.

United States Patent Application 943,443 also discloses a process for preparing activated antiperspirant salts wherein an aqueous aluminum salt solution and aluminum metal are reacted at a temperature of from 90° to 195°C. until a ratio of aluminum to anion of 0.50 - 2.5:1 is obtained. According to the present inventors, long heating times are required to aohieve activation using this process.

The patents and applications referred to above discuss chromatographic analytical techniques by which the various components and the level of activation of the salts can be determined. The technique most most suitable for this type of analysis is a form of gel permeation chromatography known as "size exclusion chromatography". Particularly good descriptions of size exclusion chromatography and the criteria used to determine the level of activation of antiperspirant salts can be found in European Patent Applications 0 256 831 and 0 256 832.

The general methods, calculations and standards for determining the activation level of an antiperspirant salt sample as presented in 0 256 831 and 0 256 832 are adopted herein as the preferred means of determining the level of activation of an antiperspirant salt and will be henceforth referred to as the "Inward Size Exclusion Chromatography Test", (or "Inward SEC") after the inventor named in the '831 and '832 patent applications, Peter Inward.

The chromatographic columns used by the applicants in their Inward SEC analysis are commercially packed and prepared columns sold under the trade name ZORBAX SEC/PSM 60-S and are available from E. I. du Pont de Nemours & Company, Wilmington, Delaware. The ZORBAX SEC/PSM 60-S columns have an internal diameter of 6.2 mm, a length of 250 mm and are packed with silylated porous silica having a pore size of 60Å. The ZORBAX SEC/PSM 60-S columns are believed to be essentially equivalent to those described in the '831 and '832 applications.

In their Inward SEC analysis, the applicants prepared the tested salt solutions so that the aluminum concentration was 2.5% by weight or less. Samples requiring dilution were mixed with .01M HCl rather than deionized water. Also, .01 M HCl was used as the chromatographic eluent rather than deionized water. Any solids in the slat solutions were filtered out before testing. The net effect of using hydrochloric acid in the Inward SEC test, rather than ionized water is negligible, as the relative retention times of the salt components remain essentially equivalent to those described in the '831 and '832 patent applications.

The typical antiperspirant salt can be readily analyzed by the Inward SEC test to determine the identity of the components comprising the antiperspirant salt and also the relative proportions of components in the salt. Of the four aluminum containing fractions eluted from a typical antiperspirant salt sample in the Inward SEC test, the species which characterize activated salts are chiefly those which are eluted as Band III on the chromatogram. The relative retention times of the aluminum containing components of an antiperspirant sample are as follows:

TABLE I

|  | BAND I | BAND II | BAND III | BAND IV |
|---|---|---|---|---|
| RELATIVE RETENTION TIME RANGE | 0.62-0.70 | 0.71-0.75 | 0.76-0.82 | 0.83-0.97 |

Whether a salt can be classified as activated is determined by the relative quantity of Band III materials as compared to the total material eluted in the Inward SEC test. The calculation used to determine the proportion of Band III materials eluted is as follows:

$$\text{Band III proportion} = 100 \times \frac{\text{Area of Band III from chromatogram}}{\text{Sum of the area of all the bands eluted except that attributed to the included species}}$$

In order to be considered activated, the Band III proportion should comprise at least 20% of the area of the chromatograph of the same eluted according to the Inward SEC testing method. In increasing order of preference, the Band III proportion should comprise at least 25% - 100% of the sample, at least 30% - 100% of the sample, at least 40% -100% of the sample, at least 80% - 100% of the sample and at least 90% - 100% of the sample when analyzed according to the Inward SEC testing method.

Although it does not appear to be critical to the process of the invention, it is preferred to maintain the Band I proportion at a low value, for example, from 0% - 5% of the sample. Higher levels of Band I content may be achieved if desired, or if a low Band I content is not of significant importance to the antiperspirant product formulator.

The present inventors have developed a process for the formation of activated antiperspirant salts, particularly of the aluminum, zirconium and aluminum/zirconium complex varieties, whereby the process comprises heating an aqueous solution of aluminum or zirconium hydrate or aluminum/zirconium hydrate mixture in the presence of Cl, Br, I, $SO_4$ or $NO_2$ for a period of time ranging from one minute to five minutes at a temperature ranging from about 140° to about 200°C. The solution of activated salt is then converted to a solid form, such as by conventional spray drying equipment.

When heated in recycling loop heat exchanger equipment, a mean heating time from one minute to thirty minutes is required, due to the back mixing of non-activated materials which naturally occurs in such processing equipment. Heating times using the recycling loop heat exchanger equipment from five to twenty minutes are preferred with heating times of five to fifteen minutes being most preferred.

While the process can be carried out batchwise, the brief heating period required for activation lends the method of this invention very readily to continuous processing techniques wherein heat exchangers and other equipment of standard design can easily accommodate the one to five minute residence time. The process of this invention, particularly when applied to a continuous process, has obvious commercial advantages over the long heating period batch techniques known in the art.

The process of the invention, in its most basic form, comprises heating an aqueous solution having the empirical formula $Z_2(OH)_{6-a}X_a$ wherein Z is a member of the group consisting of aluminum, zirconium and mixtures thereof, a ranges from 0.3 to 4 and X represents a member of the group consisting of Cl, Br, I, $SO_4$, $NO_2$ and mixtures thereof, at a temperature of from about 140° to about 220°C. for a period of time ranging from one to five minutes to thereby form an activated antiperspirant salt which, according to the Inward Size Exclusion Chromatography Test, has a Band III proportion in the range of from 20% to 100% of the area of the chromatograph of the sample eluted according to the Inward SEC testing method. The activated salt solution is dried to obtain a solid activated salt material. The drying can be carried out by any conventional means by which the resulting solid salt remains in its activated state. Such drying of the solution may be carried out by conventional spray drying equipment.

When heating is conducted in recycling loop heat exchanger equipment, a mean heating time from 1 minute to twenty minutes is required, due to the back mixing of non-activated materials which naturally occurs in such processing equipment. Heating times using the recycling loop heat exchanger equipment from five to twenty minutes are preferred with heating times of five to fifteen minutes being most preferred.

The temperature to which the salt solution is heated in the range of 140° to above 220°C., preferably in the range of from about 145° to about 220°C., more preferably from about 145° to about 200°C., and most preferably from about 160° to about 180°C. The Band III proportion preferably ranges from 25% -100%, more preferably from 30% -100%, and most preferably from 80% - 100% of the area of the chromatograph of the sample eluted according to the Inward SEC testing method. The Band I proportion preferably ranges from 0% - 5% of the area of the chromatograph of the sample eluted, but higher levels are believed to be acceptable, depending on the intended application of the antiperspirant salt.

It will be understood by those skilled in the art to which this invention pertains that $Z_{2(OH)6-a}X_a$ is an empirical formula and is intended to represent and include coordinated and/or bound water in various quantities, as well as polymers, mixtures and complexes of the above.

In another embodiment of the invention, the method of forming an activated antiperspirant salt as described hereinabove is carried out in a continuous stream process.

In yet another embodiment of the invention, an aqueous solution of $Al_2(OH)_{6-a}X_a$, wherein X and a are as defined above, is heated to a temperature ranging from about 140° to 220°C. or to the preferred ranges as set forth above for a period of time ran in from one to five minutes to activate said salt solution and that said activated salt solution is mixed directly with a zirconium halohydrate solution before drying to a solid state.

When aluminum and zirconium are both present in the antiperspirant salt solution, whether they are combined prior to or subsequent to heating of the solution to the activated state, the ratio of aluminum to zirconium atoms should be in the range of from 10:1 to 1:10, with the range of 6:1 to 1:6 being preferred, with the range of from 3:1 to 1:6 being most preferred.

In all embodiments of the invention, in order to maintain the salt material in the activated state, it is important that spray drying of the salt closely follow heating to the activated state. If the salt is permitted to cool a significant extent before being spray dried, the salt is likely to convert to a non-activated state, owing to its metastable condition when in solution. This is contrary to the teachings of those patents and published applications wherein the salt materials are allowed to cool to room temperature before being spray dried. In setting up processing equipment to practice the method of this invention, a spray dryer can be connected directly on line with the reactor or heat exchanger used for heating the materials to the activated state. In this way, the heated solution can be passed directly to the spray dryer where the solution can be converted by conventional spray drying means into a dry powdered form, allowing for greater economy in heating. Furthermore, when heated, the salt solution has a decreased viscosity, giving an added advantage in spray drying.

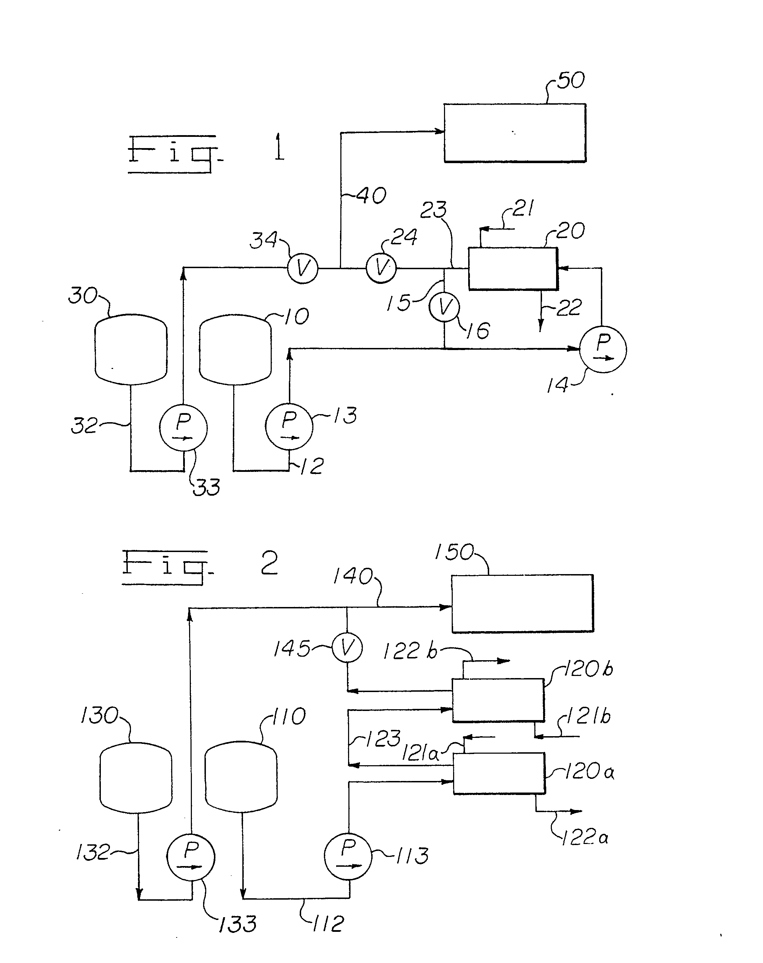

In the drawings, Figure 1 and Figure 2 are schematic representations of production equipment utilizing the method of the present invention, as applied to a continuous process for making activated antiperspirant salts.

More specifically, in Figure 1 continuous processing equipment is illustrated which features a recycling loop at the heat exchanger. A holding tank 10 feeds a stream of a raw antiperspirant salt solution, such as an aluminum halohydrate solution, via a raw feed conduit 12 to a metering pump 13 for assuring the continuous pressurized and metered flow of feed solution downstream of the tank. The raw feed conduit 12 flows to a heat exchanger 20, heated by steam or oil introduced in said heat exchanger via heat exchange media inlet 21. The heat exchange media is exited from the heat exchanger via heat exchange media outlet 22. The aluminum halohydrate solution is heated in the heat exchanger to the desired activation temperature in the range of from 140° to 220°C. Although not shown in Figure 1, it is well known in the art that to improve energy efficiency, the heating medium should be reheated and recirculated to the heat exchanger.

The hot stream of activated salt solution leaves the heat exchanger via an exit conduit 23. A preselected amount of the heated salt solution can be drawn off exit conduit 23 for recycling through the heat exchanger by means of the feed recycling conduit 15. The amount of solution recycled can be controlled by means of a value 16. A recycling pump 14, preferably a high rate pump, maximizes the velocity of the salt solution through the heat exchanger. The high velocity flow assists in the transfer of heat to the salt solution and also reduces fouling in the heat exchanger. This segment of the processing equipment of the invention is known as a "recycling loop heat exchanger".

When the recycling loop is used, as in this equipment set-up, the salt solution will not have a uniform residence time in the heat exchanger. This is due to at least part of the solution being passed from the exit conduit 23 to the spray dryer feed conduit 40 and spray dryer 50 without running through the recycling loop, whereas other portions of the solution may be passed through the recycling loop two or more times. The mean residence time of the salt solution can be readily calculated by those of ordinary skill in the art when the flow rate through the heat exchanger and the volumetric capacity of the heat exchanger are known and, according to the present invention, should be in the range of 1 minute to 5 minutes.

The portion of salt solution which passes by the feed recycling conduit 15, is conducted through a valve 24 for control of solution flow to the spray dryer feed conduit 40 and on to the spray dryer unit 50.

If desired, the activated salt solution can be mixed with a feed stream of unactivated slat solution, such as a zirconium halohydrate salt or other antiperspirant additives, held in tank 30. The materials from the additive tank 30 flows through additive conduit 32 to join the stream of activated salt materials at spray dryer feed conduit 40. A metering pump 33 and valve 34 are used to control the flow of the additive material to the activated salt stream.

Figure 2 is a schematic representation of processing equipment for practicing the continuous process of the invention featuring a "plug-flow" heat exchanger set-up. In Figure 2, a holding tank 110 feeds a stream of the raw antiperspirant solution, such as an aluminum halohydrate solution via a raw feed conduit 112 to a metering pump 113. The pump assures a continuous pressurized and metered flow of the salt solution downstream of the tank. Good processing control practices should be adhered to, to insure a stable non-varying feed rate. The stable feed rate will promote a uniformly activated product.

The stream of the raw salt solution is passed to a first stage heat exchanger 120-a where the solution is heated to a temperature in the range of from 25° to 170°C. Heat exchange media, such as steam, is introduced to the first heat exchanger via a steam inlet 121-a and the condensate of the steam is exhausted from the heat exchanger via a steam outlet 122-a.

The heated aluminum solution is passed from the first heat exchanger to the second heat exchanger 120-b via a transfer conduit 123. The second heat exchanger heats the stream of salt solution to a temperature of from 140° to 220°C. through indirect contact with a heating medium such as hot oil. The hot oil is introduced to the heat exchanger via an inlet 121-b and is exhausted from the second heat exchanger via an outlet 122-b.

The stream of hot activiated antiperspirant solution leaves the second heat exchanger via a heat exchanger exit conduit 124 where it can optionally be mixed with non-activated salt solutions and/or antiperspirant additives flowing from the additive holding tank 130, via additive conduit 132. A metering pump 133 is used to provide a measured positive flow of the additive materials to the stream of activated antiperspirant salt solution. The entire stream of materials is passed via spray dryer conduit 140 to spray dryer 150. A valve 145 or metering pump at the same location assures proper pressure and material flow to

5

the spray dryer.

At the spray dryer, the inlet temperature for the stream of materials to be dried is typically in the range of from 500 - 800°F. The outlet temperature at the spray dryer is typically in the range of from 150 - 250°F.

The concentrations of materials in the processing stream of the invention are in the range of from 10 - 50% solids content for aluminum salt solutions, with a range of 20 - 50% being preferred, 30 - 40% being more preferred and 30 - 35% being most preferred. For zirconium salts, the concentration of materials should also be in the range of from 20 - 50% solids, with 30 - 40% being preferred. When the feed mixture comprises both aluminum and zirconium salts in solution, the concentration of materials should be 10 -50% solids, with 20 - 50% being preferred and 30 - 40% being most preferred.

The pH of the solutions being processed should be maintained in the range of from about pH 2 - 5. When only aluminum salts are present, a slightly higher pH can be maintained, e.g. at pH 3 - 5, while when only zirconium salts are present, the pH can be maintained lower, e.g. at pH 2 -5.

The inventors have prepared antiperspirant materials using the process of the invention as described herein. Three process runs, Examples A, B and C, were conducted using the plug-flow process equipment as illustrated in Figure 2. The processing runs of an aluminum chlorhydrate solution were conducted under the following conditions:

EXAMPLE A

| | |
|---|---|
| Aluminum solids concentration . . . . . | 20% |
| AL/CL ratio . . . . . | 1.87:1 |
| Temperature to which solution was heated. . | 175°C. |
| Time for which solution was heated. . . . . | 3 minutes |

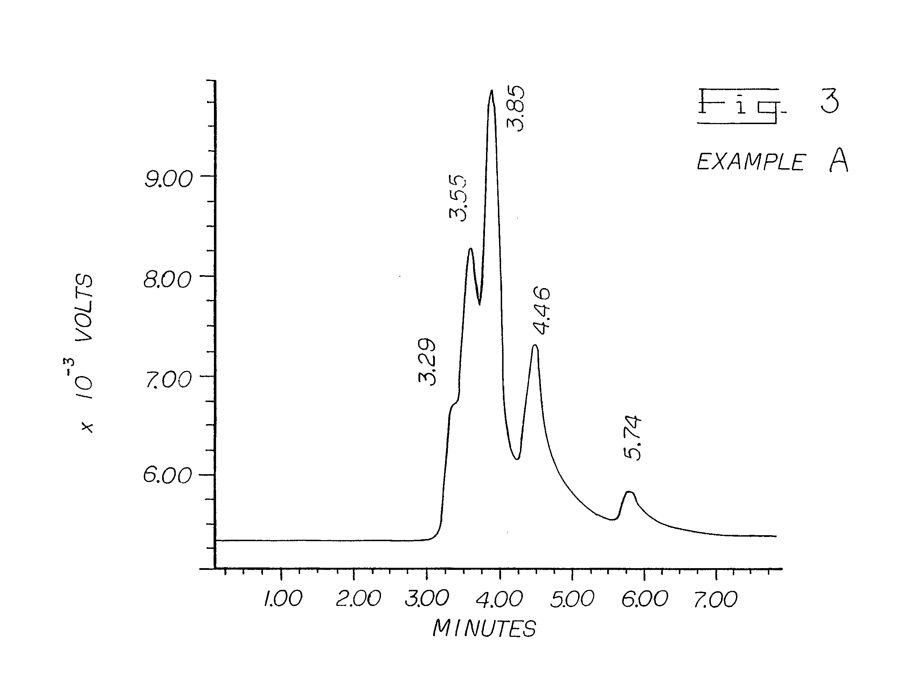

Chromatographic analysis of the activated salt produced thereby was conducted accordingly. Figure 3 is the graph of the chromatogram produced thereby showing the relative areas of the Bands.

EXAMPLE B

| | |
|---|---|
| Aluminum solids concentration . . . . . | 34% |
| AL/CL ratio . . . . . | 2:1 |
| Temperature to which solution was heated. . | 175°C. |
| Time for which solution was heated. . . . . | 3 minutes |

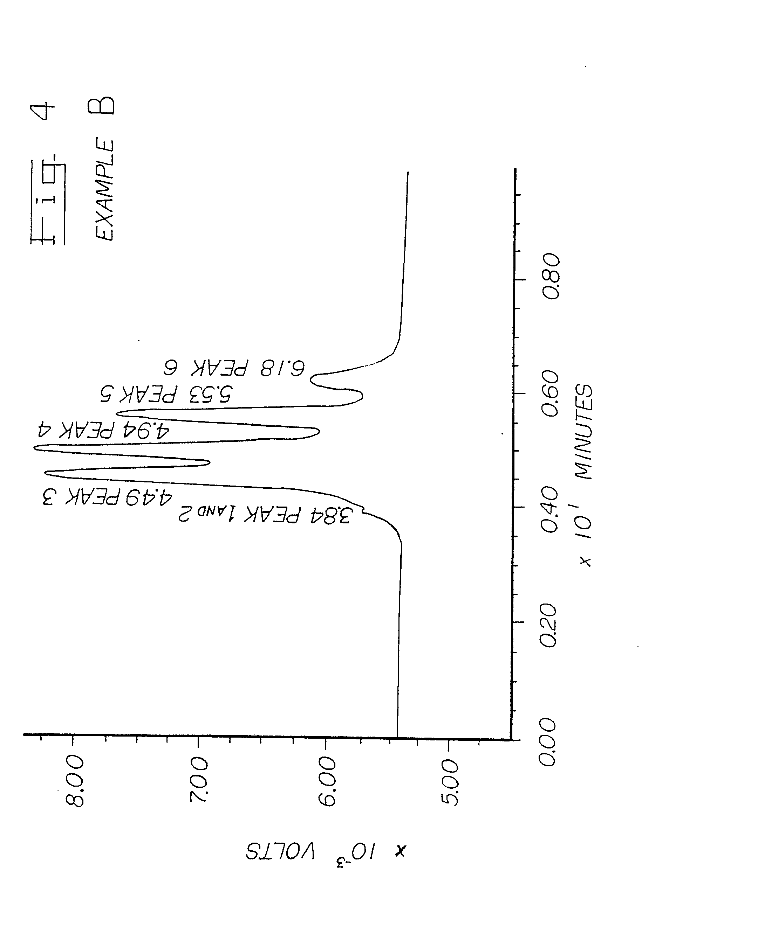

Chromatographic ananlysis of the activated salt produced thereby was conducted accordingly. Figure 4 is the graph of the chromatogram produced thereby showing the relative areas of the Bands.

EXAMPLE C

| | |
|---|---|
| Aluminum solids concentration . . . . . | 34% |
| AL/CL ratio . . . . . | 2:1 |
| Temperature to which solution was heated. . | 175°C. |
| Time for which solution was heated. . . . . | 2.1 minutes |

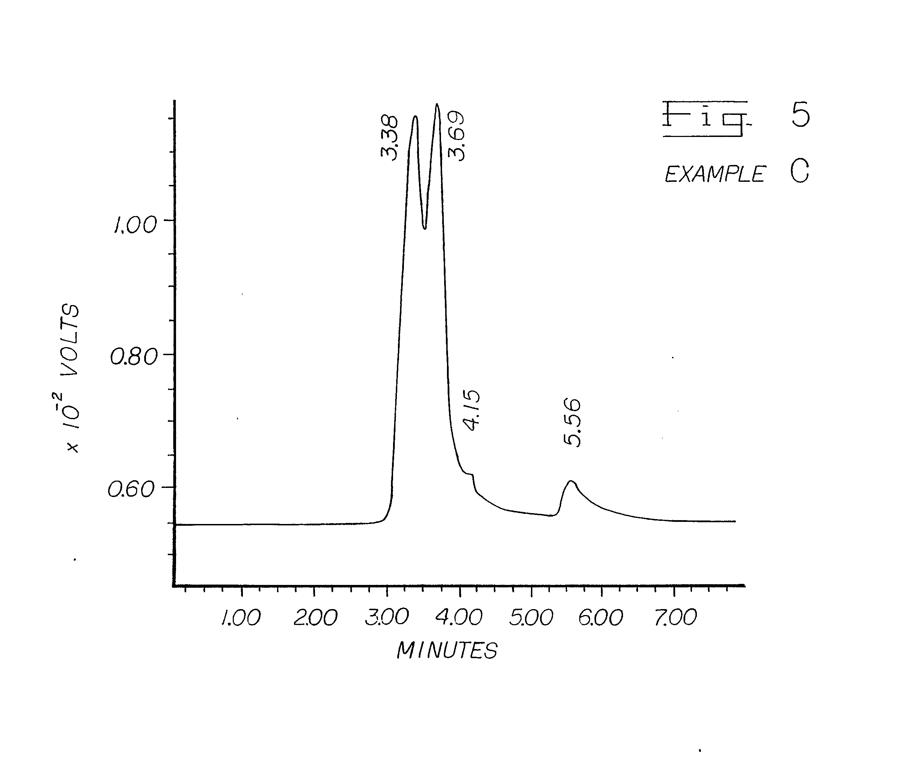

Chromatographic analysis of the activated salt produced thereby was conducted accordingly. Figure 5 is the graph of the chromatogram produced thereby showing the relative areas of the Bands.

A processing run of an aluminum chlorhydrate solution was made using the heat exchanger recycling loop processing equipment as illustrated in Figure 1.

EXAMPLE D

| Aluminum solids concentration . . . . . | 37% |
|---|---|
| AL/CL ratio . . . . . | 1.86:1 |
| Temperature to which solution was heated. . | 150° C. |
| Mean time for which solution was heated. . . . . | 3 minutes |

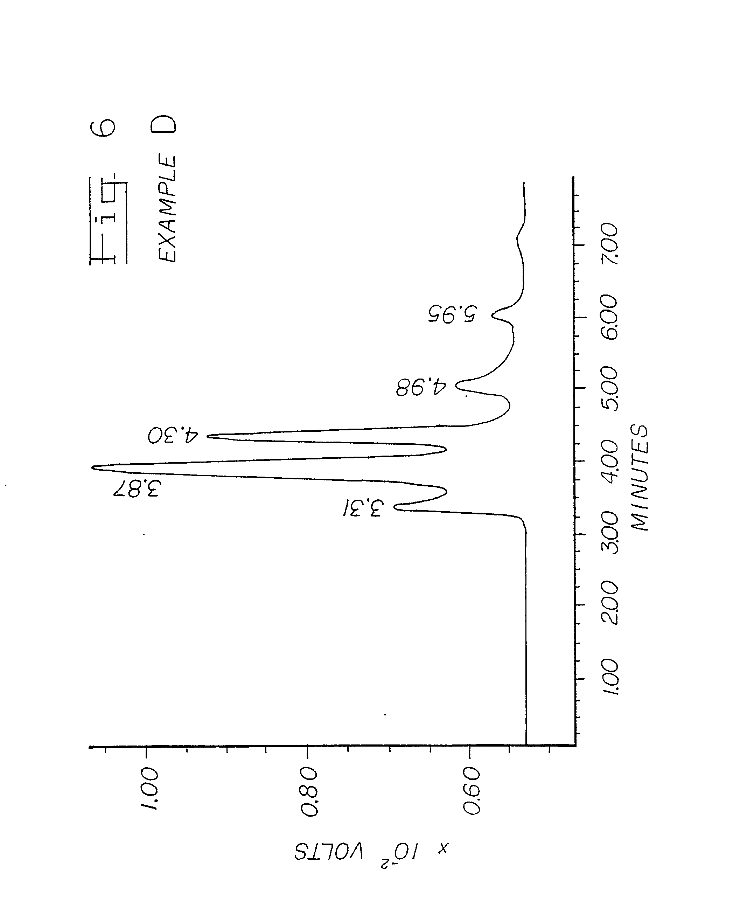

Chromatographic analysis of the activated salt produced thereby was conducted accordingly. Figure 6 is the graph of the chromatogram produced thereby showing the relative areas of the Bands.

## Claims

1. A process for the preparation of activated antiperspirant salts, wherein the process comprises heating an aqueous solution of $Z_2(OH)_{6-a}X_a$, wherein Z is a member selected from the group consisting of aluminum, zirconium and mixtures thereof, a ranges from 0.3 to 4 and X is a member selected from the group consisting of Cl, Br, I and mixtures thereof, at a temperature from about 140° to about 220° C. for a period of time ranging from one to five minutes, thereby forming an activated antiperspirant salt, which according to the Inward Size Exclusion Chromatography test, has a Band III proportion comprises from 20% to 100% of the total material of the activated salt and then spray drying the activated salt solution while said solution remains in the activated state.

2. A process as claimed in claim 1 wherein said process is continuous and said heating is carried out on a stream of $Z_2(OH)_{6-a}X_a$ solution.

3. A process as claimed in claim 1 wherein said process is conducted as a continuous stream process.

4. A process for the preparation of activated antiperspirant salts, wherein the process comprises heating an aqueous solution of $Z_2(OH)_{6-a}X_a$, wherein Z is a member selected from the group consisting of aluminum, zirconium and mixtures thereof, a ranges from 0.3 to 4 and X is a member selected from the group consisting of Cl, Br, I and mixtures thereof, at a temperature from about 140° to about 220° C. in a recycling loop heat exchanger for a mean period of time ranging from one to thirty minutes, thereby forming an activated antiperspirant salt, which according to the Inward Size Exclusion Chromatography test, has a Band III proportion comprising from 20% to 100% of the total material of the activated salt and then spray drying the activated salt solution while said solution remains in the activated state.

Fig. 1

Fig. 2

8

Fig. 3

EXAMPLE A

EP 0 405 598 A2

Fig. 4

EXAMPLE B

EP 0 405 598 A2

Fig. 5

EXAMPLE C

EP 0 405 598 A2

_Fig_ 6

EXAMPLE D

EP 0 405 598 A2